(19) Europäisches Patentamt
European Patent Office
Office européen des brevets

(11) EP 3 131 950 B1

(12) **EUROPEAN PATENT SPECIFICATION**

(45) Date of publication and mention of the grant of the patent:
**15.01.2020 Bulletin 2020/03**

(21) Application number: **15719937.3**

(22) Date of filing: **15.04.2015**

(51) Int Cl.:
***C08G 73/10*** *(2006.01)*

(86) International application number:
**PCT/US2015/025950**

(87) International publication number:
**WO 2015/160935 (22.10.2015 Gazette 2015/42)**

(54) **METHODS OF MANUFACTURE OF BIS(PHTHALIMIDE)S**

VERFAHREN ZUR HERSTELLUNG VON BIS(PHTHALIMID)EN

PROCÉDÉS DE PRODUCTION DE BIS(PHTHALIMIDE)S

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2014 US 201461979748 P**

(43) Date of publication of application:
**22.02.2017 Bulletin 2017/08**

(73) Proprietor: **SABIC Global Technologies B.V.**
**4612 PX Bergen op Zoom (NL)**

(72) Inventors:
- **CHAULAGAIN, Mani Raj**
**Mt. Vernon, Indiana 47620 (US)**
- **GUGGENHEIM, Thomas Link**
**Mt. Vernon, Indiana 47620 (US)**
- **ODLE, Roy Ray**
**Mt. Vernon, Indiana 47620 (US)**

(74) Representative: **Office Freylinger**
**P.O. Box 48**
**8001 Strassen (LU)**

(56) References cited:
**EP-A1- 2 233 512**
**CN-A- 1 560 113**
**US-A1- 2013 108 851**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

BACKGROUND

This disclosure relates to methods of manufacture of bis(phthalimide) and polyetherimide compositions.

Polyetherimides ("PEIs") are amorphous, transparent, high performance polymers having a glass transition temperature ("Tg") of greater than 180°C. PEIs further have high strength, heat resistance, modulus, and broad chemical resistance, and therefore are widely used in applications as diverse as automotive, telecommunication, aerospace, electrical/electronics, transportation, and healthcare. One process for the manufacture of polyetherimides is by polymerization of alkali metal salts of dihydroxyaromatic compounds, such as bisphenol A disodium salt ("$BPANa_2$"), with a substituted bis(phthalimide) such as a bis(halophthalimide). For example, polyetherimides can be produced by polymerization of $BPANa_2$ with 1,3-bis[N-(4-chlorophthalimido)]benzene("4-ClPAMI"), which has the following structure:

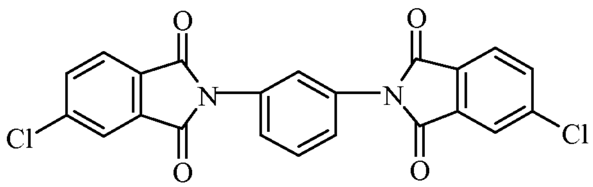

Other isomers of the ClPAMI can also be present. Substituted bis(phthalimides) such as bis(halophthalimide)s, in turn, can be produced by imidization of a substituted or halophthalic anhydride such as 3-chlorophthalic anhydride ("3-ClPA"), 4-chlorophthalic anhydride ("4-ClPA"), or mixtures thereof with an organic diamine such as m-phenylenediamine ("mPD") or p-phenylenediamine ("pPD").

The polymerization is typically carried out in the presence of a polymerization catalyst. Attempts have been made to produce polyetherimides without using any catalyst. However, such processes require the purification and isolation of substituted bis(phthalimide), which is cumbersome and not desirable in a commercial setting.

Thus there remains a need in the art for an improved process for the manufacture of polyetherimides that does not require a polymerization catalyst. It would be a further advantage if a substituted bis(phthalimide) can be made and used directly in the displacement polymerization without isolation and purification.

SUMMARY

A method for the manufacture of a bis(phthalimide) composition comprises: contacting a substituted phthalic anhydride with an organic diamine in the presence of diphenyl sulfone, sulfolane, or a combination comprising at least one of the foregoing solvents at a temperature of greater than 130°C; the contacting being conducted in the absence of an imidization catalyst; wherein the substituted phthalic anhydride has a formula

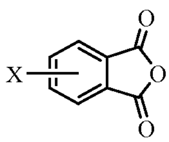

and the organic diamine has a formula

$$H_2N\text{-}R\text{-}NH_2;$$

to provide the bis(phthalimide) composition comprising diphenyl sulfone, sulfolane, or a combination comprising at least one of the foregoing solvents and a bis(phthalimide) of the formula

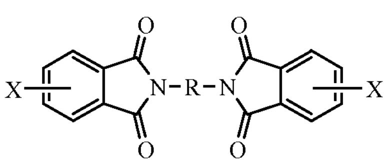

wherein, in the foregoing formulae,

X is fluoro, chloro, bromo, iodo, nitro, or a combination comprising at least one of the foregoing, and

R is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, $-(C_6H_{10})_z-$ wherein z is an integer from 1 to 4, an aromatic hydrocarbyl moiety having from 1 to 6 aromatic groups, or a divalent group of the formula

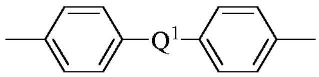

wherein $Q^1$ is a single bond, -O-, -S-,

-C(O)-, $-SO_2-$, -SO-, $-C_yH_{2y}$ wherein y is an integer from 1 to 5, or a combination comprising at least one of the foregoing,

wherein the stoichiometric molar ratio of the substituted phthalic anhydride to the organic diamine is 1.95:1 to 2.05:1.

In another embodiment, a method for the manufacture of a bis(phthalimide) composition comprises contacting a substituted phthalic anhydride with an organic diamine in the presence of diphenyl sulfone, sulfolane, or a combination comprising at least one of the foregoing solvents at a temperature of 130°C to 250°C; the contacting being conducted in the absence of an imidization catalyst; wherein the substituted phthalic anhydride has a formula

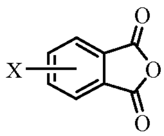

and the organic diamine has a formula

$H_2N-R-NH_2$;

to provide the bis(phthalimide) composition comprising diphenyl sulfone, sulfolane, or a combination comprising at least one of the foregoing solvents and a bis(phthalimide) of the formula

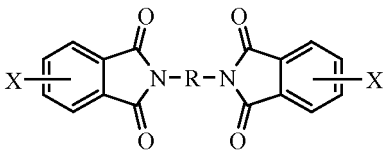

wherein, in the foregoing formulae,

X is chloro, and

R is m-phenylene, p-phenylene, arylene ether, or diarylsulfone,

wherein the stoichiometric molar ratio of the substituted phthalic anhydride to the organic diamine is 1.95:1 to 2.05:1.

In still another embodiment, a composition for manufacturing a bis(phthalimide) composition comprises a substituted phthalic anhydride and an organic diamine in sulfolane wherein the stoichiometric molar ratio of the substituted phthalic anhydride to the organic diamine is 1.95:1 to 2.05:1, the substituted phthalic anhydride has a formula

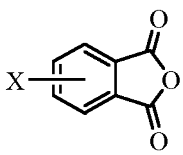

and the organic diamine has a formula

$H_2N-R-NH_2$;

wherein, in the foregoing formulae,

X is fluoro, chloro, bromo, iodo, nitro, or a combination comprising at least one of the foregoing, and
R is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, $-(C_6H_{10})_z-$ wherein z is an integer from 1 to 4, an aromatic hydrocarbyl moiety having from 1 to 6 aromatic groups, a divalent group of the formula

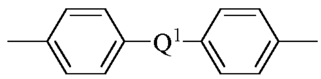

wherein $Q^1$ is a single bond, -O-, -S-, -C(O)-, $-SO_2-$, -SO-, $-C_yH_{2y}$ wherein y is an integer from 1 to 5, or a combination comprising at least one of the foregoing.

The present disclosure describes a bis(phthalimide) composition comprising diphenyl sulfone, sulfolane, or a combination comprising at least one of the foregoing solvents and a bis(phthalimide) of the formula

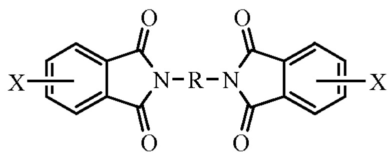

wherein

X is fluoro, chloro, bromo, iodo, nitro, or a combination comprising at least one of the foregoing, and
R is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, $-(C_6H_{10})_z-$ wherein z is an integer from 1 to 4, an aromatic hydrocarbyl moiety having from 1 to 6 aromatic groups, a divalent group of the formula

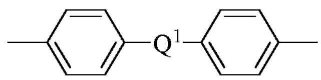

wherein $Q^1$ is a single bond, -O-, -S-, -C(O)-, $-SO_2-$, -SO-, $-C_yH_{2y}$ wherein y is an integer from 1 to 5, or a combination comprising at least one of the foregoing; and wherein the solids content is 1 to 30%, specifically 15 to 25%, preferably 18 to 22%, calculated according to the following equation.

$$\frac{\text{weight of bis(phthalimide)}}{\text{weight of bis(phthalimide)} + \text{weight of diphenyl sulphone or sulfolane}} \quad (100)$$

BRIEF DESCRIPTION OF THE DRAWINGS

A description of the figures, which are meant to be exemplary and not limiting, is provided in which:

FIG. 1 is a graph of Mw of polyetherimide as a function of polymerization time; and
FIG. 2 is a graph of Mw of polyetherimide as a function of polymerization time with different bisphenol A salt and ClPAMI molar ratio.

DETAILED DESCRIPTION

The inventor hereof has surprisingly found that it is now possible to make substituted bis(phthalimide)s in polar aprotic solvents having a high boiling point, for example, diphenyl sulfone or tetramethylene sulfone, without using any

imidization catalyst. The prepared substituted bis(phthalimide)s in polar aprotic solvents can be used directly in displacement polymerization without separating it from the polar aprotic solvents. In an advantageous feature, no polymerization catalyst is required for the polymerization.

Polyetherimides produced by the methods disclosed herein have formula (1)

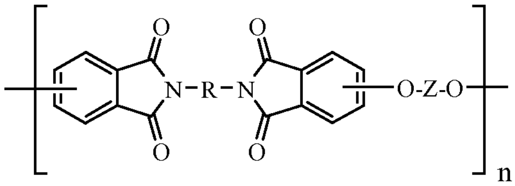

(1)

wherein n is greater than 1, for example 10 to 1,000, specifically 10 to 500 or 10 to 100, preferably 10 to 50.

The group R in formula (1) is a $C_{6-27}$ aromatic hydrocarbon group or a halogenated derivative thereof, a straight or branched chain $C_{2-20}$, specifically $C_{2-10}$ alkylene group or a halogenated derivative thereof, a $C_{3-20}$ cycloalkylene group or halogenated derivative thereof, $-(C_6H_{10})_z-$ wherein z is an integer from 1 to 4, an aromatic hydrocarbyl moiety having from 1 to 6 aromatic groups, and a group of formula (2)

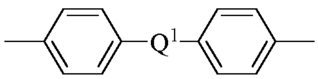

(2)

wherein $Q^1$ is -O-, -S-, -C(O)-, $-SO_2-$, -SO-, $-C_yH_{2y}-$ wherein y is an integer from 1 to 5, or a combination comprising at least one of the foregoing.

In an embodiment R is $-(C_6H_{10})_z-$ wherein z is an integer from 1 to 4 or a divalent group of formulae (3)

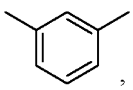 , 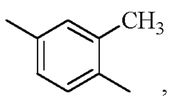 , 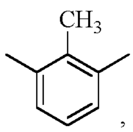 , 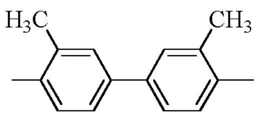 , 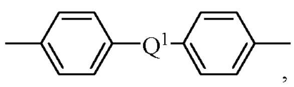 ,

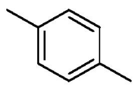 , 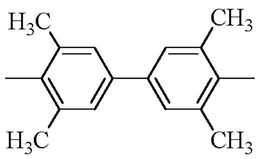 , 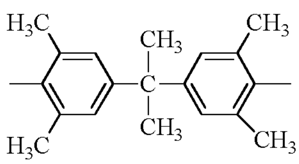 , 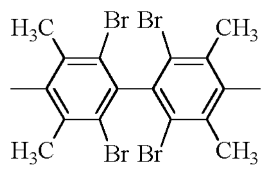 ,

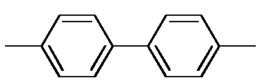 , 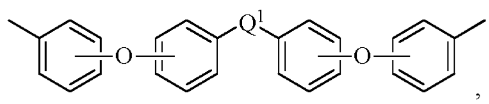 , 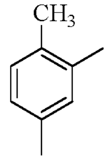

(3)

wherein $Q^1$ is -O-, -S-, -C(O)-, $-SO_2-$, -SO-, $-C_yH_{2y}-$ or halogenated derivatives thereof wherein y is an integer from 1 to 5, or a combination comprising at least one of the foregoing. In some embodiments, R is the diether aromatic moiety of formula (3) having four phenylene groups wherein $Q^1$ is a direct bond, -O-, -S-, -C(O)-, $-SO_2-$, -SO-, or $-C_yH_{2y}-$ or halogenated derivatives thereof wherein y is an integer from 1 to 5. In some embodiments R is m-phenylene, p-phenylene, or diarylsulfone. The diarylsulfone can be, for example, 4,4'-diphenylsulfone. Embodiments where R is a divalent arylene ether can also be specifically mentioned, for example an arylene ether of the formula

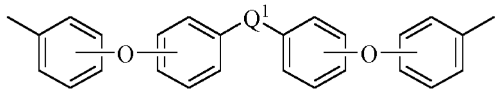

(3a)

wherein $Q^1$ is a direct bond, -O-, -S-, -C(O)-, $-SO_2-$, -SO-, $-C_yH_{2y}-$ or halogenated derivatives thereof wherein y is an integer from 1 to 5, or a combination comprising at least one of the foregoing. In an embodiment, $Q^1$ in formula (3a) is -O-.

The group Z in formula (1) is a substituted or unsubstituted divalent organic group, and can be an aromatic $C_{6-24}$ monocyclic or polycyclic moiety optionally substituted with 1 to 6 $C_{1-8}$ alkyl groups, 1 to 8 halogen atoms, or a combination thereof, provided that the valence of Z is not exceeded. Exemplary groups Z include groups of formula (4):

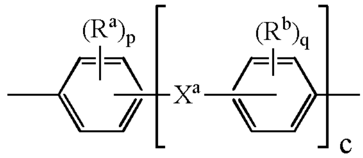

(4)

wherein $R^a$ and $R^b$ are each independently a halogen atom or a monovalent hydrocarbon group and can be the same or different; p and q are each independently integers of 0 to 4; c is 0 to 4, specifically zero or 1; and $X^a$ is a bridging group connecting the two aromatic groups, where the bridging group and point of attachment of each $C_6$ arylene group are disposed ortho, meta, or para (specifically para) to each other on the $C_6$ arylene group. The bridging group $X^a$ can be a single bond, -O-, -S-, -S(O)-, $-S(O)_2-$, -C(O)-, or a $C_{1-18}$ organic bridging group. The $C_{1-18}$ organic bridging group can be cyclic or acyclic, aromatic or non-aromatic, and can further comprise heteroatoms such as halogens, oxygen, nitrogen, sulfur, silicon, or phosphorous. The $C_{1-18}$ organic group can be disposed such that the $C_6$ arylene groups connected thereto are each connected to a common alkylidene carbon or to different carbons of the $C_{1-18}$ organic bridging group. A specific example of a group Z is a divalent group of formula (4a)

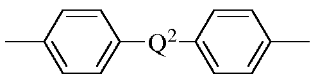

(4a)

wherein $Q^2$ is a single bond, -O-, -S-, -C(O)-, $-SO_2-$, -SO-, $-C_yH_{2y}-$ or a halogenated derivative thereof wherein y is an integer from 1 to 5, including perfluoroalkylene groups. In a specific embodiment $Q^2$ is 2,2-isopropylidene. In another specific embodiment $Q^2$ is a single bond.

In another specific embodiment, the polyetherimide comprises more than 1, specifically 10 to 100, 10 to 80, or 10 to 50, structural units of formula (1) wherein R is a divalent group of formula (2) wherein $Q^1$ is $-C_yH_{2y}-$ wherein y is an integer from 1 to 5, and Z is a group of formula (4a) wherein $Q^2$ is -O-, -S-, -C(O)-, $-SO_2-$, -SO-, and $-C_yH_{2y}-$ or a halogenated derivative thereof wherein y is an integer from 1 to 5. In some embodiments, R is m-phenylene, p-phenylene, p,p-diphenylether, diphenylsulfone, or a combination comprising at least one of the foregoing, and Z is 2,2-(4-phenylene)isopropylidene. In some embodiments, the polyetherimide is a polyetherimide sulfone. A specific polyetherimide sulfone comprises structural units of formula (1) wherein at least 50 mole percent of the R groups are of formula (4a) wherein $Q^2$ is $-SO_2-$ and the remaining R groups are independently p-phenylene or m-phenylene or a combination comprising at least one of the foregoing; and Z is 2,2-(4-phenylene)isopropylidene.

The polyetherimides are prepared first by imidization of a substituted phthalic anhydride with an organic diamine to form a bis(phthalimide), followed by polymerization of the bis(phthalimide) at the substituted position. In this method, a substituted phthalic anhydride of formula (7)

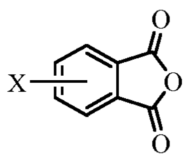

(7)

wherein X is a leaving group (such as a nitro group or a halogen), is condensed (imidized) with an organic diamine of formula (8)

$$H_2N\text{-}R\text{-}NH_2 \qquad (8)$$

wherein R is as described in formula (1), in the presence of diphenyl sulfone at a temperature of greater than 130°C to form a composition comprising bis(phthalimide) of formula (9) and diphenyl sulfone, sulfolane, or a combination comprising at least one of the foregoing solvents

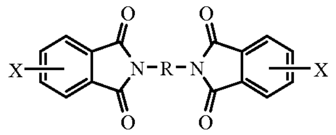

(9)

wherein X is a leaving group as in formula (7) and R is a linker as described in formula (1).

In an embodiment, X is a nitro group or a halogen, specifically fluoro, chloro, bromo, iodo, more specifically chloro. A mixture of different X groups can be used.

Illustrative examples of amine compounds of formula (8) include ethylenediamine, propylenediamine, trimethylenediamine, diethylenetriamine, triethylenetetramine, hexamethylenediamine, heptamethylenediamine, octamethylenediamine, nonamethylenediamine, decamethylenediamine, 1,12-dodecanediamine, 1,18-octadecanediamine, 3-methylheptamethylenediamine, 4,4-dimethylheptamethylenediamine, 4-methylnonamethylenediamine, 5-methylnonamethylenediamine, 2,5-dimethylhexamethylenediamine, 2,5-dimethylheptamethylenediamine, 2, 2-dimethylpropylenediamine, N-methyl-bis (3-aminopropyl) amine, 3-methoxyhexamethylenediamine, 1,2-bis(3-aminopropoxy) ethane, bis(3-aminopropyl) sulfide, 1,4-cyclohexanediamine, bis-(4-aminocyclohexyl) methane, m-phenylenediamine, p-phenylenediamine, 2,4-diaminotoluene, 2,6-diaminotoluene, m-xylylenediamine, p-xylylenediamine, 2-methyl-4,6-diethyl-1,3-phenylene-diamine, 5-methyl-4,6-diethyl-1,3-phenylene-diamine, benzidine, 3,3'-dimethylbenzidine, 3,3'-dimethoxybenzidine, 1,5-diaminonaphthalene, bis(4-aminophenyl) methane, bis(2-chloro-4-amino-3, 5-diethylphenyl) methane, bis(4-aminophenyl) propane, 2,4-bis(b-amino-t-butyl) toluene, bis(p-b-amino-t-butylphenyl) ether, bis(p-b-methyl-o-aminophenyl) benzene, bis(p-b-methyl-o-aminopentyl) benzene, 1, 3-diamino-4-isopropylbenzene, bis(4-aminophenyl) ether and 1,3-bis(3-aminopropyl) tetramethyldisiloxane. Mixtures of these amines can be used. Illustrative examples of amine compounds of formula (8) containing sulfone groups include diamino diphenyl sulfone (DDS) and bis(aminophenoxy phenyl) sulfones (BAPS). Combinations comprising any of the foregoing amines can be used.

Specifically, diamine (8) is a meta-phenylene diamine (8a), a para-phenylene diamine (8b), or a diamino diaryl sulfone (8c), or an arylene ether (8d)

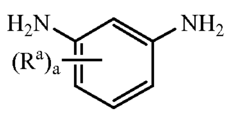

(8a)

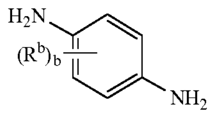

(8b)

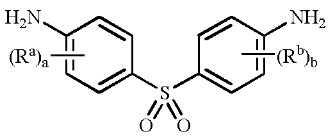

(8c)

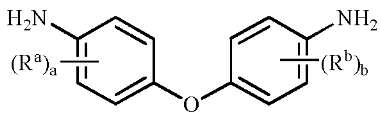

(8d)

wherein $R^a$ and $R^b$ are each independently a halogen atom, nitro, cyano, $C_2$-$C_{20}$ aliphatic group, $C_2$-$C_{40}$ aromatic group, and a and b are each independently 0 to 4. Specific examples include meta-phenylenediamine (mPD), para-phenylenediamine (pPD), 2,4-diaminotoluene, 2,6-diaminotoluene, 2-methyl-4,6-diethyl-1,3-phenylenediamine, 5-methyl-4,6-diethyl-1,3-phenylenediamine, 1,3-diamino-4-isopropylbenzene, 4,4'-oxydianiline and 4,4'-diamino diphenyl sulfone. In some embodiments of bis(phthalimide) (9), X is chloro or fluoro, specifically chloro, and R is m-phenylene, p-phenylene, arylene ether, a diarylsulfone, or a combination thereof.

The substituted phthalic anhydride of formula (7), the organic diamine of formula (8), and diphenyl sulfone, sulfolane, or a combination comprising at least one of the foregoing solvents can be combined in any order. In an embodiment, the diphenyl sulfone is first heated to a molten state, then the substituted phthalic anhydride of formula (7), the organic diamine of formula (8) are added to the molten diphenyl sulfone either alone, or in combination.

In an advantageous feature, the condensation of a substituted phthalic anhydride of formula (7) and an organic diamine of formula (8) (imidization) is conducted without any imidization catalysts typically used in imidization reactions.

The bis(phthalimide)s (9) are generally prepared at least at 130°C, specifically 150° to 275°C, more specifically 160 to 250°C. Atmospheric or super-atmospheric pressures can be used, for example up to 5 atmospheres, to facilitate the use of high temperatures without causing solvent to be lost by evaporation.

The reaction of the substituted phthalic anhydride (7) with the organic diamine (8) to form bis(phthalimide) (9) is generally conducted for 0.5 to 30 hours, specifically 1 to 20 hours, more specifically 1 to 10 hours, still more specifically 2 to 8 hours, and yet more specifically 3 to 7 hours. Advantageously, conversion to the bis(phthalimide) is 99% complete, based on the moles of the substituted phthalic anhydride, in less than 6 hours.

The diphenyl sulfone, sulfolane, or a combination comprising at least one of the foregoing solvents, organic

diamine (8) and substituted phthalic anhydride (7) can be combined in amounts such that the total solids content during the reaction to form bis(phthalimide) (9) does not exceed 80 weight percent (wt.%), or does not exceed 60 wt.%. For example, the total solids content can be 1 to 40 wt.%, or 1 to 30 wt.%, or 1 to 25 wt.%. "Total solids content" expresses the proportion of the reactants as a percentage of the total weight including liquids such as molten diphenyl sulfone, sulfolane, or a combination comprising at least one of the foregoing solvents present in the reaction at any given time.

In an embodiment, the amount of diphenyl sulfone or sulfolane is adjusted based on the amount of bis(phthalimide) (9). In particular, the amount is based on the weight of the bis(phthalimide) (9) divided by the sum of the weight of the bis(phthalimide) (9) plus the weight of the diphenyl sulfone or sulfolane as follows:

$$\frac{\text{weight of bis(phthalimide) (9)}}{\text{weight of bis(phthalimide) (9) + weight of diphenyl sulphone or sulfolane}}(100)$$

and can be 1 to 30%, specifically 15 to 25%, or 18 to 22%.

It can be desirable to have low water content in the imidization reaction mixture. Thus, in some embodiments, the combined substituted phthalic anhydride, organic diamine, and diphenyl sulfone, sulfolane, or a combination comprising at least one of the foregoing solvents can comprise less than or equal to 200 parts per million parts of the combined components weight (ppm) of water, more specifically, less than or equal to 100 ppm of water, still more specifically, less than or equal to 50 ppm of water, or, yet more specifically, less than or equal to 25 ppm of water. In some embodiments, the combined substituted phthalic anhydride, organic diamine, and diphenyl sulfone, sulfolane, or a combination comprising at least one of the foregoing solvents comprise less than or equal to 100 ppm water.

A molar ratio of substituted phthalic anhydride (7) to diamine (8) of 1.95:1 to 2.05:1, specifically 1.98:1 to 2.02:1, more specifically 1.98:1 to 2.01:1, or 2:1, can be used. While other ratios can be employed, a slight excess of anhydride or diamine can be desirable. A proper stoichiometric balance between substituted phthalic anhydride (7) and diamine (8) is maintained to prevent undesirable by-products that can limit the molecular weight of the polyetherimide polymer prepared from the bis(phthalimide), and/or result in polymers with amine end groups. Accordingly, in an embodiment, the imidization process includes contacting diamine (8) with substituted phthalic anhydride (7) in the presence of diphenyl sulfone, sulfolane, or a combination comprising at least one of the foregoing solvents at a temperature of greater than 130°C to form a reaction mixture having a targeted initial molar ratio of substituted phthalic anhydride to diamine; further heating the reaction mixture; analyzing the molar ratio of the further heated reaction mixture to determine the actual initial molar ratio of substituted phthalic anhydride (7) to diamine (8); and, if necessary, adding substituted phthalic anhydride (7) or diamine (8) to the analyzed reaction mixture to adjust the molar ratio of substituted phthalic anhydride (7) to diamine (8) to the desired value, for example 1.98:1 to 2.02:1 or other ratio as described above. The imidization reaction can be conducted in the presence of a catalyst or in the absence of a catalyst.

In some embodiments, an endcapping agent is formed during imidization, or the imidization is conducted in the presence of an endcapping agent. The endcapping agent can be formed before or during imidization by addition of a monofunctional reactant that reacts with one of the amine groups of diamine (8), thereby "capping" the amine end group. Other endcapping agents include phenates, for example the salts of any monophenol, p-cumyl phenol. Such mono-capped diamines endcap the polymer during polymerization, and thus can be used to control the molecular weight of the polymer or the end groups of the polymer. Accordingly, the monofunctional reactant has a functional group that reacts with an amine of diamine (8), for example, a phthalic anhydride, acyl alkyl halide, acyl aryl halide, aldehyde, ketone, ester, isocyanate, chloroformate, sulfonyl chloride, a $C_{1-12}$ primary amine, and the like. A combination of different monofunctional reactants can be present. In an embodiment the monofunctional reactant is a phthalic anhydride without a halogen, nitrogen, or other leaving group substitution. For example, when a combination of substituted phthalic anhydride (7) and unsubstituted phthalic anhydride are reacted with organic diamine (8), the product comprises bis(phthalimide) (9) and monofunctional bis(phthalimide) (18)

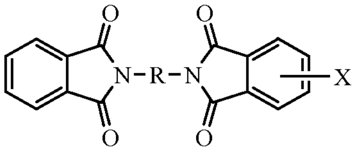

(18)

wherein R and X are as defined in formula (9).

The amount of monofunctional reactant added depends on the desired amount of endcapping agent. For example, the amount of monofunctional reactant present in the imidization reaction can be more than 0 to 10 mole

percent, specifically 1 to 10 mole percent, and more specifically 0.1 to 6 mole percent, based on total moles of endcapping agent and substituted phthalic anhydride (7). The monofunctional reactant can be added at any time, e.g., to the diamine (8), the substituted phthalic anhydride (7), the solvent, or a combination thereof, before or after imidization has started, in the presence or absence of the imidization catalyst.

Alternatively, or in addition to the above monofunctional reactants, monofunctional monofunctional bis(phthalimides) can be added directly as endcapping agents. Thus, in some embodiments, the method further comprises the addition of an endcapping agent such as a monofunctional bis(phthalimide) (18) or monofunctional phthalimide (19). Thus, imidization can be conducted by stepwise, simultaneously or essentially simultaneously combining the reactants, i.e., substituted phthalic anhydride (7), organic amine (8), solvent, imidization catalyst, and the monofunctional reactant or endcapping agent such as a monofunctional bis(phthalimide).

The bis(phthalimide) composition comprising diphenyl sulfone, sulfolane, or a combination comprising at least one of the foregoing solvents can be used for the subsequent polymerization step, described below, without purification. Alternatively, the bis(phthalimide) composition can be subject to further purification as is known in the art before polymerization.

Thus, after imidization, the leaving group X of bis(phthalimide) (9)

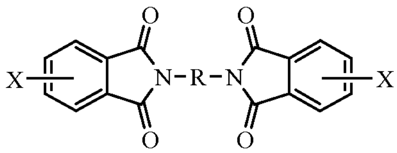

(9)

is displaced by reaction with an alkali metal salt of a dihydroxy aromatic compound of formula (10)

MO-Z-OM (10)

wherein M is an alkali metal and Z is as described in formula (1), to provide the polyetherimide of formula (1)

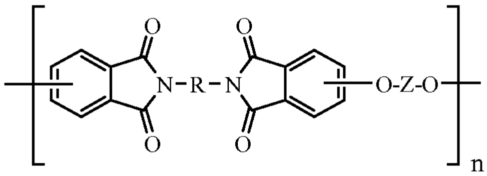

(1)

wherein n, R, and Z are as defined above.

Alkali metal M can be any alkali metal, for example lithium, sodium, potassium, and cesium. Thus alkali metal salt (10) is lithium salts, sodium salts, potassium salts, cesium salts, or a combination thereof. Specific alkali metals are potassium or sodium. In some embodiments, M is potassium. The alkali metal salt can be obtained by reaction of a metal hydroxide with aromatic $C_{6-24}$ monocyclic or polycyclic dihydroxy aromatic compound optionally substituted with 1 to 6 $C_{1-8}$ alkyl groups, 1 to 8 halogen atoms, or a combination thereof, for example a dihydroxy aromatic compound of formula (11):

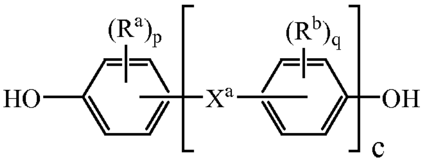

(11)

wherein $R^a$, $R^b$, and $X^a$ are as described in formula (4). In some embodiments the dihydroxy aromatic compound is bisphenol A, hydroquinone, bisphenol, resorcinol, or a combination comprising at least one of the foregoing. For example, the dihydroxy aromatic compound can be 2,2-bis(4-hydroxyphenyl) propane ("bisphenol A" or "BPA"). The alkali metal salt can be used in a powder form or in a slurry form. The slurry form of the alkali metal salt comprises an alkali metal salt dispersed in a solvent such as ortho-dichlorobenzene, toluene, molten diphenyl sulfone, sulfolane, and xylenes. Once combined with bis(phthalimide) (9), the solvent in the slurry of alkali metal salt can be removed before the polymerization reaction starts. In an embodiment, the slurry form comprises an alkali metal salt dispersed in ortho-dichlorobenzene.

Advantageously, polymerization of bis(phthalimide) (9) in diphenyl sulfone, sulfolane, or a combination comprising at least one of the foregoing solvents with alkali metal salt (10) can be conducted without the presence of a polymerization catalyst. In another embodiment, the polymerization is conducted in the presence of a polymerization catalyst. Examples of polymerization catalysts are the quaternary ammonium salts, quaternary phosphonium salts, guanadinium salts, pyridinium salts, imidazolium salts described above, in particular guanidinium salts. Examples of guanidinium salts are hexaalkylguanidinium and $\alpha,\omega$(pentaalkylguanidinium)alkane salts, and an example of a hexaalkylguanidinium salt is hexaethylguanidinium chloride.

If necessary, additional diphenyl sulfone, sulfolane, or a combination comprising at least one of the foregoing solvents can be added. Other non-polar solvent, preferably with a boiling point above 100°C, specifically above 150°C, for example o-dichlorobenzene, dichlorotoluene, 1,2,4-trichlorobenzene, a monoalkoxybenzene such as anisole, veratrole, diphenylether, or phenetole, or a polar aprotic solvent such as dimethylformamide (DMF), dimethylacetamide (DMAc), dimethylsulfoxide (DMSO), tetramethylene sulfone (sulfolane), and N-methylpyrrolidinone (NMP) can be used as a cosolvent. In an embodiment, the polymerization is carried out in diphenyl sulfone and no other solvents are used. A total solids content of the bis(phthalimide) (9) in the polymerization can be 15 to 25 wt.%, based on the total weight of the polymerization mixture. "Total solids content" refers to the proportion of the reactants as a percentage of the total weight of the polymerization mixture, including solvents, such as diphenyl sulfone, sulfolane, or a combination comprising at least one of the foregoing solvents.

Polymerization can be conducted at least 110°C, specifically 150° to 275°C, more specifically 160 to 250°C. Atmospheric or super-atmospheric pressures can be used, for example up to 5 atmospheres, to facilitate the use of high temperatures without causing solvent to be lost by evaporation.

The polymerization can be conducted for 0.5 to 30 hours, specifically 1 to 20 hours, more specifically 1 to 10 hours, still more specifically 2 to 8 hours, and yet more specifically 3 to 7 hours. The yellowness index of polyetherimide (1) can depend on the polymerization time such that the longer the polymerization time, the higher the yellowness index. Thus it is generally desirable to minimize the polymerization time.

In some embodiments, the alkali metal salt (10) is added directly to the composition containing the bis(phthalimide) (9) and the diphenyl sulfone, sulfolane, or a combination comprising at least one of the foregoing solvents. Water removal from the system can be accomplished in either batch, semi-continuous or continuous processes using means known in the art such as a distillation column in conjunction with one or more reactors. Other methods for water removal include passing the condensed distillate through a drying bed for chemical or physical adsorption of water.

The molar ratio of the alkali metal salt (10) to bis(phthalimide) (9) can be 0.9:1 to 0.999:1, specifically 0.95:1 to 0.99:1.

The bis(phthalimide) composition and the polyetherimide composition can be manufactured in separate vessels. Advantageously, since the bis(phthalimide) composition can be used directly in the polymerization reaction without purification, the bis(phthalimide) composition and the polyetherimide composition can be manufactured in the same vessel. As described above, in some embodiments an endcapping agent is formed during imidization or added to the imidization. Alternatively, the polymerization further comprises the addition of an endcapping agent. The endcapping agent can be the monofunctional bis(phthalimide) (18), monofunctional phthalimide (19). Monofunctional endcapping agents (18) and (19) react with the alkali metal phenoxide end groups present in the polymerization step. Other endcapping agents reactive with the alkali metal dihydroxy aromatic compound (10) can be used. The amount of the endcapping agent can vary. In an embodiment, for instance, the amount can be 1 to 2 mole equivalents, per mole equivalent of excess alkali metal dihydroxy aromatic compound (10) present in the system. The endcapping agent can be added stepwise, simultaneously or essentially simultaneously with the bis(phthalimide) (9) and the alkali metal salt (10).

The polyetherimides can be formulated to provide a wide variety of polyetherimide compositions for the manufacture of articles. The polyetherimide compositions can optionally comprise a filler. In some instances it is desired to have polyetherimide compositions wherein a filler is substantially absent. "Substantially absent" means that the composition has less than 3 wt.% of a filler, and in other embodiments less than 1 wt.% filler by weight of the composition. In other instances, it is advantageous to have polyetherimide compositions wherein a filler is absent.

The polyetherimide compositions can include various additives ordinarily incorporated into polymer compositions of this type, with the proviso that the additives are selected so as to not significantly adversely affect the desired properties of the composition. Exemplary additives include catalysts, impact modifiers, fillers, antioxidants, thermal stabilizers, light stabilizers, ultraviolet light (UV) absorbing additives, quenchers, plasticizers, lubricants, mold release agents, antistatic agents, visual effect additives such as dyes, pigments, and light effect additives, flame retardants, anti-drip agents, and radiation stabilizers. In some embodiments, the polyetherimide composition comprises a solvent, and the composition is in the form of a varnish. Combinations of additives can be used, for example a combination of a heat stabilizer, a mold release agent, and optionally an ultraviolet light stabilizer. In general, the additives are used in the amounts generally known to be effective. The foregoing additives (except any fillers) are generally present in an amount

from 0.005 to 20 wt.%, specifically 0.01 to 10 wt.%, based on the total weight of the composition. Alternatively, in some embodiments, our compositions do not contain appreciable amounts of additives, and in some embodiments, there are no detectable amounts of additives, i.e., additives are substantially absent or absent from the compositions. Accordingly, the foregoing additives (except any fillers) can be present in an amount from 0 to 20 wt.%, 0 to 15 wt.%, 0 to 10 wt.%, or 0.0001 to 20 wt.%, based on the total weight of the composition. In another embodiment, no appreciable amount of any additive other than a heat stabilizer, a mold release agent, and optionally an ultraviolet light stabilizer is present in the compositions. In still another embodiment, no detectable amount of any additive other than a heat stabilizer, a mold release agent, and optionally an ultraviolet light stabilizer is present in the compositions.

In some embodiments, the polyetherimide composition can further include at least one additional polymer. Examples of such additional polymers include and are not limited to PPSU (polyphenylene sulfone), polyetherimides, PSU (polysulfone), PPE (polyphenylene ether), PFA (perfluoroalkoxy alkane), MFA (co-polymer of TFE tetrafluoroethylene and PFVE perfluorinated vinyl ether), FEP (fluorinated ethylene propylene polymers), PPS (poly(phenylene sulfide), PTFE (polytetrafluoroethylene), PA (polyamide), PBI (polybenzimidizole), PAI (poly(amide-imide)), poly(ether sulfone), poly(aryl sulfone), polyphenylene, polybenzoxazoles, polybenzthiazoles, as well as blends and co-polymers thereof. When present, the polymer is used in an amount from more than 0 to 20 wt.%, specifically 0.1 to 15 wt.%, and more specifically from 0.5 to 10 wt.%, all based on the total weight of the polyetherimide composition. In some embodiments, no polymer other than the polyetherimide as described herein is present in the polyetherimide composition.

The polyetherimide composition can be prepared by blending the ingredients under conditions for the formation of an intimate blend. Such conditions often include melt mixing in single or twin screw-type extruders, mixing bowl, or similar mixing devices that can apply a shear to the components. Twin-screw extruders are often preferred due to their more intensive mixing capability and self-wiping capability, over single screw extruders. It is often advantageous to apply a vacuum to the blend through at least one vent port in the extruder to remove volatile impurities in the composition. Often it is advantageous to dry the polyetherimide composition prior to melt mixing. The melt mixing is often done at 290 to 340°C to avoid excessive polymer degradation while still allowing sufficient melting to get an intimate polymer mixture free of any unbelted components. The polymer blend can also be melt filtered using a 40 to 100 micrometer candle or screen filter to remove undesirable black specks or other heterogeneous contaminants.

In an exemplary process, the polyetherimide, any other polymers, and any additives are placed into an extrusion compounder to produce a continuous strand that is cooled and then chopped into pellets. In another procedure, the components are mixed by dry blending, and then fluxed on a mill and comminuted, or extruded and chopped. The composition and any optional components can also be mixed and directly molded, e.g., by injection or transfer molding techniques. Preferably, all of the components are freed from as much water as possible. In addition, compounding is carried out to ensure that the residence time in the machine is short; the temperature is carefully controlled; the friction heat is utilized; and an intimate blend between the components is obtained.

The polyetherimide composition can be formed into an article by any number of methods including shaping, extruding (including profile extrusion), thermoforming, and molding, including injection molding, compression molding, gas assist molding, structural foam molding, and blow molding. In some embodiments, a method of forming an article comprises shaping, extruding, blow molding, or injection molding the composition to form the article. The polyetherimide compositions can also formed into articles using thermoplastic processes such as film extrusion, sheet extrusion, melt casting, blown film extrusion, and calendaring. Co-extrusion and lamination processes can be used to form composite multi-layer films or sheets. The article is a sheet, film, multilayer sheet, multilayer film, molded part, extruded profile, coated part, pellets, powder, foam, fiber, fibrids, flaked fibers, or a combination comprising at least one of the foregoing.

The polyetherimide composition can be molded into an article with any equipment conventionally used for molding thermoplastic compositions, such as a Newbury or van Dorn type injection-molding machine with conventional cylinder temperatures of 250°C to 320°C, and conventional mold temperatures of 55°C to 120°C.

It is appreciated that in an embodiment, tetramethylene sulfone (sulfolane) can be used as an alternative to diphenyl sulfone. Accordingly, whenever diphenyl sulfone is mentioned, it can be replaced with tetramethylene sulfone (sulfolane).

The methods of the manufacture of bis(phthalimide) and polyetherimide compositions are further illustrated by the following non-limiting examples.

## EXAMPLES

### Materials

The materials in Table 1 were used or made in the following Examples and Comparative Examples.

Table 1

| Acronym | Description | Source |
|---|---|---|
| BPA | 2,2-Bis(4-hydroxyphenyl)propane, (Bisphenol A) | Hexion |
| $K_2BPA$ | Bisphenol, dipotassium salt | Examples |
| $Na_2BPA$ | Bisphenol, disodium salt | Examples |
| KOH | Potassium hydroxide | Acculute |
| IPA | Isopropyl alcohol | Aldrich |
| DPS | Diphenyl sulfone | |
| DDS | Diaminodiphenyl sulfone | |
| o-DCB | ortho-Dichlorobenzene | Fischer |
| ClPA | Mixture of 3-chlorophthalic anhydride and 4-chlorophthalic anhydride | SABIC |
| 3C1PA | 3-Chlorophthalic anhydride | |
| 4C1PA | 4-Chlorophthalic anhydride | |
| mPD | meta-Phenylene diamine | DuPont |
| pPD | para-Phenylene diamine | |
| ODA | Oxydianiline | |
| AcOH | Acetic acid | Aldrich |
| ClPAMI | 1,3-bis[N-(3- or 4-chlorophthalimido)]benzene | Examples |
| 3ClPAMI | 1,3- or 1,4-bis[N-(3-Chlorophthalimido)]benzene | Examples |
| 4ClPAMI | 1,3- or 1,4-bis[N-(4-Chlorophthalimido)]benzene | Examples |
| 4ClPAMI-DPE | 4,4'-bis[N-(4-Chlorophthalimido)]diphenyl ether | Examples |
| 3ClPAMI-DPE | 4,4'-bis[N-(3-Chlorophthalimido)]diphenyl ether | Examples |
| 3ClPAMI-DPS | 4,4'-bis[N-(3-Chlorophthalimido)]diphenyl sulfone | Examples |
| 4ClPAMI-DPS | 4,4'-bis[N-(4-Chlorophthalimido)]diphenyl sulfone | Examples |
| PA | Phthalic anhydride | |
| NaPCP | Sodium para-cumyl phenol | |
| HEGCl | Hexaethyl guanidinium chloride | |
| PEI | Polyetherimide | Examples |
| $H_3PO_4$ | Phosphoric acid | Fischer |

PROPERTY TESTING

Weight average molecular weight (Mw) of the polymer product was determined by gel permeation chromatography (GPC) using polystyrene standards.

In a 20 ml glass vial, about 20 mg of the polymer sample was taken and dissolved into a quench solution (3.5 L $CH_2Cl_2$ + 120 mL AcOH + 30 mL *o*-DCB) followed by filtration with 0.25 micrometer filter into an HPLC vial. The solution was analyzed by GPC with polystyrene standard (HPLC 2695, Waters GPC software using 2487 Dual absorbance detector of wavelength 254 nm and Mixed Bed C, PLgel 5 micrometers, 300 x 7.5 mm, P/N 1110-6500 column).

$BPAK_2$ powder

A 500 mL 3-neck round bottomed flask (24/40) was equipped with an overhead stirrer through its center joint. One of the side joints was connected to a nitrogen sweep while the other was connected to a nitrogen blanket connected to a bubbler via a Dean-Stark trap with its arm wrapped in a heating tape. The flask was then charged with 11.4145 g BPA (0.05 moles, 1 equiv.) and 0.1 moles aqueous KOH solution. The overhead stirrer was turned on and the flask was

immersed into the oil bath at 80°C. The stirring was continued for 1h. Another 500 mL 3-neck flask with the above set-up was charged with 200 mL xylenes and heated to 140°C. The aqueous salt solution was slowly cannulated into the flask containing the heated xylenes and xylenes/water was stripped off into the Dean Stark trap. After removing majority of the water, the salt precipitated out as solid on the wall of the flask. The temperature of the flask was decreased to 100°C and 100 mL isopropanol was added to the flask. The solid dissolved again forming a solution. Upon stripping of the solvents while slowly increasing the temperature to 150°C, the solution started to become cloudy. After IPA and the remaining water were removed, a $K_2$BPA salt slurry in xylenes was formed. The salt was converted into a dry powder by further stripping off xylenes. The power was dried in a vacuum oven at 140°C for 12 h.

BPA$Na_2$ slurry

A 500 mL 3-neck round bottomed flask (24/40) was equipped with an overhead stirrer through its center joint. One of the side joints was connected to a nitrogen sweep while the other was connected to a nitrogen blanket connected to a bubbler via a Dean-Stark trap with its arm wrapped in a heating tape. The flask was then charged with 11.4145 g BPA (0.05 moles, 1 equiv.) and 0.1 moles aqueous KOH solution (Acculute). The overhead stirrer was turned on and the flask was immersed into the oil bath at 80°C. The stirring was continued for 1 hour. Another 500 mL 3-neck flask with the above set-up was charged with 200 mL o-DCB and heated to 160°C. The aqueous salt solution was slowly cannulated into the flask with o-DCB. The water and o-DCB was stripped off into the Dean-Stark with the nitrogen sweep while the salt slurry was forming. The stripping off continued until the Karl-Fisher analysis of the overheads condensate showed the moisture level <50 ppm.

Example 1.

This example demonstrates the synthesis of ClPAMI from ClPA and mPD in diphenyl sulfone without using other solvents.

A 500 mL 3-neck round bottomed flask (24/40) was equipped with an overhead stirrer through its center joint. One of the side joints was connected to a nitrogen sweep while the other was connected to a nitrogen blanket connected to a bubbler via a Dean-Stark trap with its arm wrapped in a heating tape. The flask was then immersed into an oil bath at 170°C and DPS (50 g) was added. Once the DPS was completely molten, stirrer was turned on and 2.671 g *m*-PD (0.0247 moles, 1.0 equiv.) and 9.054 g ClPA (0.0496 moles, 2.008 equiv.) were added into the molten DPS (making it ~18% solid) at 170°C and the temperature was slowly increased to 200°C. The initial solution converted into a thick white slurry of ClPAMI. The heating was continued for 3 h and the slurry became slightly thinner. At that point, the stoichiometry analysis of the reaction mixture showed the presence of 0.66 mol % of residual 4ClPA and 0.61 mol% of residual monoamine in the reaction mixture.

Example 2

This example demonstrates that the ClPAMI composition containing diphenyl sulfone can be reacted with BPA$K_2$ powder directly without purification. No catalyst is needed for the polymerization.

To the stirring slurry of ClPAMI (10.8 g, 0.0247 moles, 1 equiv.) from Example 1, $K_2$BPA salt powder (7.308 g, 95.7 % solid, 0.024 moles, 0.93 equiv.) was added. The temperature of the reaction mixture was increased from 170°C to 220°C. The mixture first became thick solid and then became thinner. Mw build was monitored by GPC analysis. Additional $K_2$BPA salt was added to adjust the molar ratio of $K_2$BPA to ClPAMI to 0.95 (2.5 h) and 0.97 (5.5 h) respectively. The Mw of polyetherimide as a function of time is shown in FIG. 1. The reaction was quenched with phosphoric acid (85%, 670 mg) at 170°C and stirred for 30 min. The mixture was then transferred into a 500 mL flask with a Teflon cap and cooled. Methylene chloride (200 mL) was added into the solidified polymer solution. The mixture was shaken to convert the solid into a suspension. The suspension was filtered through 2.7 micrometer filter paper in a Buchner Funnel to remove the precipitated solid. The clear polymer solution in DPS and methylene chloride was slowly added to 300 mL acetone with constant agitation by a homogenizer to precipitate the polyetherimide which was filtered and washed with 200 mL acetone to provide a polyetherimide powder, which was subsequently dried in vacuum at room temperature.

Example 3

This example demonstrates that the ClPAMI composition containing diphenyl sulfone can be reacted with BPA$Na_2$ slurry directly without purification. No catalyst is needed for the polymerization.

A slurry of BPA$Na_2$ in o-DCB made in a separate flask was added to the ClPAMI slurry made in example 1. Then o-DCB was stripped off into Dean-Stark with the help of heating tape and nitrogen sweep. Once ODCB was removed, polymerization started. The Mw was monitored in GPC with polystyrene standard. Decreasing the molar ratio

of salt and ClPAMI decreased the Mw of polyetherimide, showing the possibility of Mw control. The results are shown in FIG. 2. Polyetherimide was quenched and isolated following the same procedure as shown in Example 3.

Example 4

Following the procedure described in example 1, 5.409 g m-PD (0.05 moles, 1.0 equiv.) and 18.329 g 4ClPA (0.1004 moles, 2.008 equiv.) were used to synthesize 4ClPAMI. The residual levels of 4ClPA and monoamine were 0.31 and 0.34 mol% respectively.

Example 5

Following the procedure described in example 1, 5.409 g m-PD (0.5 moles, 1.0 equiv.) and 18.237 g 4ClPA (0.0999 moles, 1.998 equiv.) and 74 mg PA (0.05 mmol, 0.01 equiv) were used to synthesize 4ClPAMI. The residual levels of 4ClPA and monoamine were 0.67 and 0.31 mol% respectively.

Example 6

Following the procedure described in example 1, 5.409 g m-PD (0.05 moles, 1.0 equiv.) and 18.329 g 3ClPA (0.1004 moles, 2.008 equiv.) were used to synthesize 3ClPAMI. The residual levels of 3ClPA and monoamine were 0.74 and 0.31 mol% respectively.

Example 7

Following the procedure described in example 1, 5.409 g pPD (0.05 moles, 1.0 equiv.) and 18.329 g 3ClPA (0.1004 moles, 2.008 equiv.) were used to synthesize 3ClPAPI. The residual levels of 3ClPA and monoamine were 0.67 and 0.22 mol% respectively.

Example 8

Following the procedure described in example 1, 5.409 g pPD (0.05 moles, 1.0 equiv.) and 18.329 g 4ClPA (0.1004 moles, 2.008 equiv.) were used to synthesize 4ClPAPI. The residual levels of 4ClPA and monoamine were 0.55 and 0.9 mol% respectively.

Example 9

Following the procedure described in example 1, 10.012 g ODA (0.05 moles, 1.0 equiv.) and 18.329 g 3ClPA (0.1004 moles, 2.008 equiv.) were used to synthesize 3ClPAMIDPE.

Example 10

Following the procedure described in example 1, 10.012 g ODA (0.05 moles, 1.0 equiv.) and 18.329 g 4ClPA (0.1004 moles, 2.008 equiv.) were used to synthesize 4ClPAMIDPE.

Example 11

Following the procedure described in example 1, 12.415 g 4,4'-diaminodiphenyl sulfone, DDS (0.05 moles, 1.0 equiv.) and 18.329 g 3ClPA (0.1004 moles, 2.008 equiv.) were used to synthesize 3ClPAPI-DPS.

Example 12

Following the procedure described in example 1, 12.415 g 4,4'-diaminodiphenyl sulfone, DDS (0.05 moles, 1.0 equiv.) and 18.329 g 4ClPA (0.1004 moles, 2.008 equiv.) were used to synthesize 4ClPAMI-DPS.

Example 13

A 500 mL 3-neck round bottomed flask (24/40) was equipped with an overhead stirrer. The flask was also connected to a nitrogen sweep and a nitrogen blanket. The nitrogen blanket was connected to a bubbler via a Dean-Stark trap with its arm wrapped in a heating tape. The flask was charged with 5.409 g m-PD (0.05 moles, 1.0 equiv.)

and 18.329 g 4ClPA (0.1004 moles, 2.008 equiv.) followed by the addition of warm sulfolane (making it ~18% solid) at 170°C and the temperature was slowly increased to 200°C. The initial solution converted into a thick white slurry of 4ClPAMI in sulfolane. The heating was continued for 4 h and the slurry became slightly thinner. At that point, the stoichiometry analysis of the reaction mixture showed the presence of 0.82 mol % of residual 4ClPA and 0.94 mol% of residual monoamine in the reaction mixture.

Example 14

Following the procedure described in example 13, 5.409 g *m*-PD (0.05 moles, 1.0 equiv.) and 18.329 g 3-ClPA (0.1004 moles, 2.008 equiv.) were used to synthesize 3ClPAMI.

Example 15

Following the procedure described in example 13, 5.409 g pPD (0.05 moles, 1.0 equiv.) and 18.329 g 3ClPA (0.1004 moles, 2.008 equiv.) were used to synthesize 3ClPAPI.

Example 16

Following the procedure described in example 13, 10.02 g 4,4'-ODA (0.05 moles, 1.0 equiv.) and 18.329 g 3ClPA (0.1004 moles, 2.008 equiv.) were used to synthesize 3ClPAMIDPE.

Example 17

Following the procedure described in example 13, 10.02 g 4,4'-ODA (0.05 moles, 1.0 equiv.) and 18.329 g 4ClPA (0.1004 moles, 2.008 equiv.) were used to synthesize 4ClPAMIDPE.

Example 18

Following the procedure described in example 13, 10.02 g 4,4'-ODA (0.05 moles, 1.0 equiv.), 12.779 g 4ClPA (0.07 moles, 1.4 equiv.) and 5.477 g 3ClPA (0.03 moles, 0.6 equiv) were used to synthesize 4,4'-bis[N-(3,4-Chlorophthalimido)]diphenyl ether 3,4'-ClPAMI-DPE.

The invention is further illustrated by the following embodiments, which are non-limiting.

Embodiment 1: A method for the manufacture of a bis(phthalimide) composition, the method comprising contacting a substituted phthalic anhydride with an organic diamine in the presence of diphenyl sulfone, sulfolane, or a combination comprising at least one of the foregoing solvents at a temperature of greater than 130°C; the contacting being conducted in the absence of an imidization catalyst; wherein the substituted phthalic anhydride has a formula

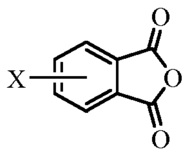

and the organic diamine has a formula $H_2N\text{-}R\text{-}NH_2$; to provide the bis(phthalimide) composition comprising diphenyl sulfone, sulfolane, or a combination comprising at least one of the foregoing solvents, and a bis(phthalimide) of the formula

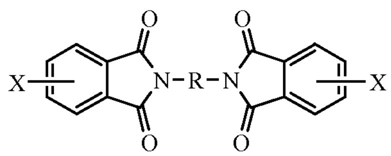

wherein, in the foregoing formulae, X is fluoro, chloro, bromo, iodo, nitro, or a combination comprising at least one of the foregoing, and R is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, $-(C_6H_{10})_z-$ wherein z is an

integer from 1 to 4, an aromatic hydrocarbyl moiety having from 1 to 6 aromatic groups, a divalent group of the formula

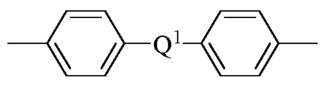

wherein $Q^1$ is a single bond, -O-, -S-, -C(O)-, $-SO_2-$, -SO-, $-C_yH_{2y}$ wherein y is an integer from 1 to 5, or a combination comprising at least one of the foregoing, wherein the stoichiometric molar ratio of the substituted phthalic anhydride to the organic diamine is 1.95:1 to 2.05:1.

Embodiment 2: The method of Embodiment 1, wherein the stoichiometric molar ratio of the substituted phthalic anhydride to the organic diamine is 1.98:1 to 2.02:1

Embodiment 3: The method of Embodiment 1 or Embodiment 2, wherein a solids content is 1 to 30%, specifically 15 to 25%, preferably 18 to 22%, calculated according to the following equation.

$$\frac{\text{weight of bis(phthalimide)}}{\text{weight of bis(phthalimide)} + \text{weight of diphenyl sulphone or sulfolane}} \ (100)$$

Embodiment 4: The method of any one or more of Embodiments 1 to 3, further comprising determining the stoichiometric molar ratio of the substituted phthalic anhydride to the organic diamine during the imidization of the substituted phthalic anhydride and the organic diamine; and optionally adjusting the stoichiometric molar ratio by adding additional substituted phthalic anhydride or organic diamine.

Embodiment 5: The method of any one or more of Embodiments 1 to 4, further comprising heating the diphenyl sulfone to a temperature of greater than 130°C, and combining the substituted phthalic anhydride and the organic diamine with the heated diphenyl sulfone.

Embodiment 6: The method of any one or more of Embodiments 1 to 5, further comprising adding a monofunctional reactant to the substituted phthalic anhydride, the organic diamine, a solvent, or a combination comprising at least one of the foregoing, wherein the solvent comprises diphenyl sulfone, sulfolane, or a combination comprising at least one of the foregoing.

Embodiment 7: The method of any one or more of Embodiments 1 to 6, wherein the contacting is conducted at a temperature of 130°C to 250°C.

Embodiment 8: The method of any one or more of Embodiments 1 to 7, wherein the contacting is conducted in the absence of an imidization catalyst.

Embodiment 9: The method of any one or more of Embodiments 1 to 7, wherein the contacting is conducted in the presence of an imidization catalyst.

Embodiment 10: The method of any one or more of Embodiments 1 to 9, wherein X is chloro, fluoro, bromo, or nitro, and R is a divalent radical of the formula - $(C_6H_{10})_z$- wherein z is an integer from 1 to 4,

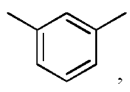 , 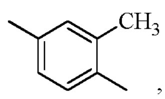 , 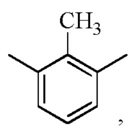 , 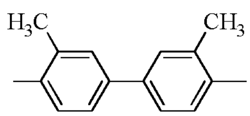 , 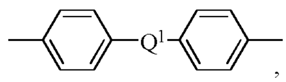 ,

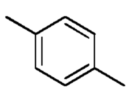 , 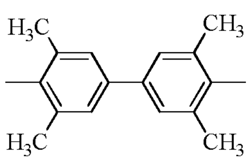 , 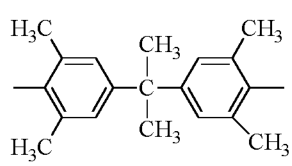 , 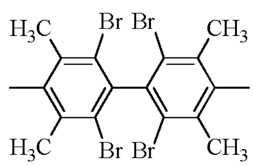 ,

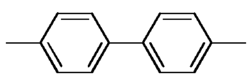 , 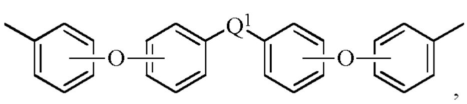 , 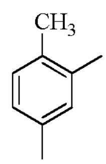

or a combination comprising at least one of the foregoing, wherein $Q^1$ is a single bond, -O-, - S-, -C(O)-, $-SO_2-$, -SO-, $-C_yH_{2y}-$ wherein y is an integer from 1 to 5, or a combination comprising at least one of the foregoing.

Embodiment 11: The method of any one or more of Embodiments 1 to 10, wherein X is chloro and R is m-phenylene, p-phenylene, diarylsulfone, a group of the formula

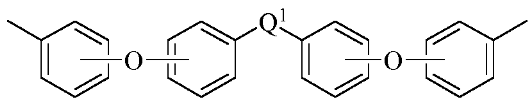

wherein $Q^1$ is a single bond, -O-, -S-, -C(O)-, $-SO_2-$, -SO-, $-C_yH_{2y}$ wherein y is an integer from 1 to 5, or a combination comprising at least one of the foregoing.

Embodiment 12: A method for the manufacture of a bis(phthalimide) composition, the method comprising contacting a substituted phthalic anhydride with an organic diamine in the presence of diphenyl sulfone, sulfolane, or a combination comprising at least one of the foregoing solvents at a temperature of 130°C to 250°C; the contacting being conducted in the absence of an imidization catalyst; wherein the substituted phthalic anhydride has a formula

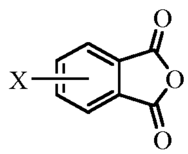

and the organic diamine has a formula $H_2N\text{-}R\text{-}NH_2$; to provide the bis(phthalimide) composition comprising diphenyl sulfone, sulfolane, or a combination comprising at least one of the foregoing solventss and a bis(phthalimide) of the formula

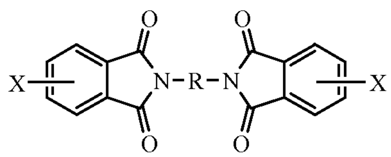

wherein, in the foregoing formulae, X is chloro, and R is m-phenylene, p-phenylene, arylene ether, or diarylsulfone, wherein the stoichiometric molar ratio of the substituted phthalic anhydride to the organic diamine is 1.98:1 to 2.02:1.

Embodiment 13: The method of Embodiment 13 or Embodiment 14, wherein

$$\frac{\text{weight of bis(phthalimide)}}{\text{weight of bis(phthalimide)} + \text{weight of diphenyl sulphone or sulfolane}}(100)$$

is 1 to 30%, specifically 15 to 25%, preferably 18 to 22%.

Embodiment 14: The method of Embodiment 12 or 13, further comprising determining the stoichiometric molar ratio of the substituted phthalic anhydride to the organic diamine during the imidization of the substituted phthalic anhydride and the organic diamine; and optionally adjusting the stoichiometric molar ratio by adding additional substituted phthalic anhydride or organic diamine.

Embodiment 15: The method of any one or more of Embodiments 12 to 14, further comprising heating the diphenyl sulfone to a temperature of 130°C to 250°C, and combining the substituted phthalic anhydride and the organic diamine with the heated diphenyl sulfone.

Embodiment 16: The method of any one or more of Embodiments 12 to 14, further comprising adding a monofunctional reactant to the substituted phthalic anhydride, the organic diamine, a solvent, or a combination comprising at least one of the foregoing, wherein the solvent comprises diphenyl sulfone, sulfolane, or a combination comprising at least one of the foregoing.

Embodiment 17: The method of any one or more of Embodiments 12 to 16, wherein the contacting is conducted in the absence of an imidization catalyst.

Embodiment 18: A composition for manufacturing a bis(phthalimide) composition, comprising a substituted phthalic anhydride and an organic diamine in sulfolane wherein the stoichiometric molar ratio of the substituted phthalic

anhydride to the organic diamine is 1.95:1 to 2.05:1, the substituted phthalic anhydride has a formula

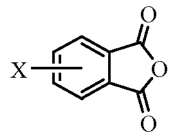

and the organic diamine has a formula $H_2N-R-NH_2$; wherein, in the foregoing formulae, X is fluoro, chloro, bromo, iodo, nitro, or a combination comprising at least one of the foregoing, and R is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, $-(C_6H_{10})_z-$ wherein z is an integer from 1 to 4, an aromatic hydrocarbyl moiety having from 1 to 6 aromatic groups, a divalent group of the formula

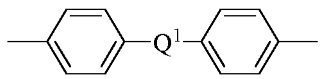

wherein $Q^1$ is a single bond, -O-, -S-, -C(O)-, $-SO_2-$, -SO-, $-C_yH_{2y}$ wherein y is an integer from 1 to 5, or a combination comprising at least one of the foregoing.

The singular forms "a," "an," and "the" include plural referents unless the context clearly dictates otherwise. "Or" means "and/or." The endpoints of all ranges directed to the same component or property are inclusive and independently combinable. Unless defined otherwise, technical and scientific terms used herein have the same meaning as is commonly understood by one of skill in the art to which this invention belongs. As used herein, a "combination" is inclusive of blends, mixtures, alloys, reaction products, and the like.

Compounds are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group.

As used herein, the term "hydrocarbyl" includes groups containing carbon, hydrogen, and optionally one or more heteroatoms (e.g., 1, 2, 3, or 4 atoms such as halogen, O, N, S, P, or Si). "Alkyl" means a branched or straight chain, saturated, monovalent hydrocarbon group, e.g., methyl, ethyl, i-propyl, and n-butyl. "Alkylene" means a straight or branched chain, saturated, divalent hydrocarbon group (e.g., methylene ($-CH_2-$) or propylene ($-(CH_2)_3-$)). "Alkenyl" and "alkenylene" mean a monovalent or divalent, respectively, straight or branched chain hydrocarbon group having at least one carbon-carbon double bond (e.g., ethenyl ($-HC=CH_2$) or propenylene ($-HC(CH_3)=CH_2-$). "Alkynyl" means a straight or branched chain, monovalent hydrocarbon group having at least one carbon-carbon triple bond (e.g., ethynyl). "Alkoxy" means an alkyl group linked via an oxygen (i.e., alkyl-O-), for example methoxy, ethoxy, and sec-butyloxy. "Cycloalkyl" and "cycloalkylene" mean a monovalent and divalent cyclic hydrocarbon group, respectively, of the formula $-C_nH_{2n-x}$ and $-C_nH_{2n-2x}-$ wherein x is the number of cyclization(s). "Aryl" means a monovalent, monocyclic or polycyclic aromatic group (e.g., phenyl or naphthyl). "Arylene" means a divalent, monocyclic or polycyclic aromatic group (e.g., phenylene or naphthylene). The prefix "halo" means a group or compound including one more halogen (F, Cl, Br, or I) substituents, which can be the same or different. The prefix "hetero" means a group or compound that includes at least one ring member that is a heteroatom (e.g., 1, 2, or 3 heteroatoms, wherein each heteroatom is independently N, O, S, or P.

Unless otherwise indicated, each of the foregoing groups can be unsubstituted or substituted, provided that the substitution does not significantly adversely affect synthesis, stability, or use of the compound. The term "substituted" as used herein means that at least one hydrogen on the designated atom or group is replaced with another group, provided that the designated atom's normal valence is not exceeded. When the substituent is oxo (i.e., =O), then two hydrogens on the atom are replaced. Combinations of substituents and/or variables are permissible provided that the substitutions do not significantly adversely affect synthesis or use of the compound. Groups that can be present on a substituted position include ($-NO_2$), cyano (-CN), hydroxy (-OH), halogen, thiol (-SH), thiocyano (-SCN), $C_{2-6}$ alkanoyl (e.g., acyl ($H_3CC(=O)-$); carboxamido; $C_{1-6}$ or $C_{1-3}$ alkyl, cycloalkyl, alkenyl, and alkynyl (including groups having at least one unsaturated linkages and from 2 to 8, or 2 to 6 carbon atoms); $C_{1-6}$ or $C_{1-3}$ alkoxy; $C_{6-10}$ aryloxy such as phenoxy; $C_{1-6}$ alkylthio; $C_{1-6}$ or $C_{1-3}$ alkylsulfinyl; C1-6 or $C_{1-3}$ alkylsulfonyl; aminodi($C_{1-6}$ or $C_{1-3}$)alkyl; $C_{6-12}$ aryl having at least one aromatic rings (e.g., phenyl, biphenyl, naphthyl, or the like, each ring either substituted or unsubstituted aromatic); $C_{7-19}$ arylalkyl having 1 to 3 separate or fused rings and from 6 to 18 ring carbon atoms; or arylalkoxy having 1 1 to 3 separate or fused rings and from 6 to 18 ring carbon atoms.

## Claims

1. A method for the manufacture of a bis(phthalimide) composition, the method comprising contacting a substituted phthalic anhydride with an organic diamine in the presence of diphenyl sulfone, sulfolane, or a combination comprising at least one of the foregoing solvents at a temperature of greater than 130°C; the contacting being conducted in the absence of an imidization catalyst;
wherein the substituted phthalic anhydride has a formula

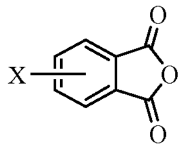

and the organic diamine has a formula

$$H_2N\text{-}R\text{-}NH_2;$$

to provide the bis(phthalimide) composition comprising diphenyl sulfone, sulfolane, or a combination comprising at least one of the foregoing solvents, and a bis(phthalimide) of the formula

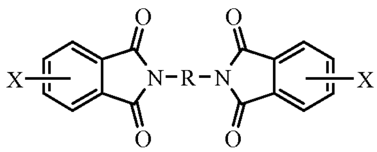

wherein, in the foregoing formulae,

X is fluoro, chloro, bromo, iodo, nitro, or a combination comprising at least one of the foregoing, and
R is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, $-(C_6H_{10})_z-$ wherein z is an integer from 1 to 4, an aromatic hydrocarbyl moiety having from 1 to 6 aromatic groups, a divalent group of the formula

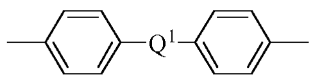

wherein $Q^1$ is a single bond, -O-, -S-,
-C(O)-, $-SO_2-$, -SO-, $-C_yH_{2y}$ wherein y is an integer from 1 to 5, or a combination comprising at least one of the foregoing,
wherein the stoichiometric molar ratio of the substituted phthalic anhydride to the organic diamine is 1.95:1 to 2.05:1.

2. The method of Claim 1, wherein the method comprises contacting the substituted phthalic anhydride and the organic diamine in the presence of sulfolane at a temperature of greater than 130°C to provide the bis(phthalimide) composition comprising sulfolane and the bis(phthalimide).

3. The method of Claim 1 or Claim 2, wherein the stoichiometric molar ratio of the substituted phthalic anhydride to the organic diamine is 1.98:1 to 2.02:1.

4. The method of any one of more of Claims 1 to 3, wherein

$$\frac{\text{weight of bis(phthalimide)}}{\text{weight of bis(phthalimide)} + \text{weight of diphenyl sulphone or sulfolane}} (100)$$

is 1 to 30%, specifically 15 to 25%, preferably 18 to 22%.

5. The method of any one or more of Claims 1 to 4, further comprising
determining the stoichiometric molar ratio of the substituted phthalic anhydride to the organic diamine during the imidization of the substituted phthalic anhydride and the organic diamine; and
optionally adjusting the stoichiometric molar ratio by adding additional substituted phthalic anhydride or organic diamine.

6. The method of any one or more of Claims 1, and 3 to 5, further comprising heating the diphenyl sulfone to a temperature of greater than 130°C, and combining the substituted phthalic anhydride and the organic diamine with the heated diphenyl sulfone.

7. The method of any one or more of Claims 1 to 6, further comprising adding a monofunctional reactant to the substituted phthalic anhydride, the organic diamine, a solvent, or a combination comprising at least one of the foregoing, wherein the solvent comprises diphenyl sulfone, sulfolane, or a combination comprising at least one of the foregoing.

8. The method of any one or more of Claims 1 to 7, wherein the contacting is conducted at a temperature of 130°C to 250°C.

9. The method of any one or more of Claims 1 to 8, wherein
X is chloro, fluoro, bromo, or nitro, and
R is a divalent radical of the formula $-(C_6H_{10})_z-$ wherein z is an integer from 1 to 4,

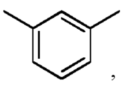 , 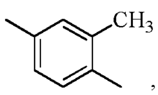 , 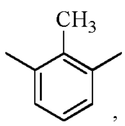 , 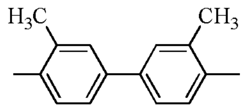 , 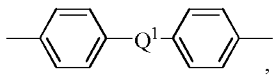 ,

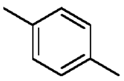 , 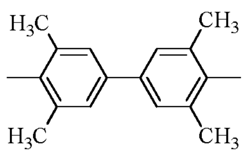 , 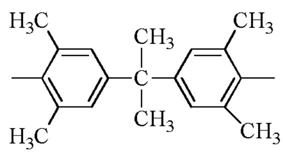 , 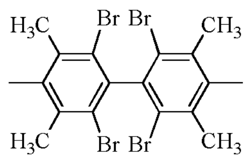 ,

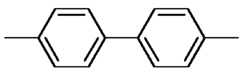 , 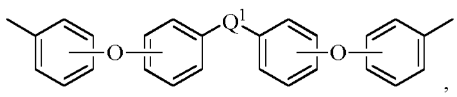 , 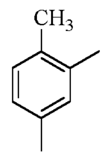

or a combination comprising at least one of the foregoing, wherein $Q^1$ is a single bond, -O-, -S-, - C(O)-, $-SO_2-$, -SO-, $-C_yH_{2y}-$ wherein y is an integer from 1 to 5, or a combination comprising at least one of the foregoing.

10. The method of any one or more of Claims 1 to 9, wherein X is chloro and R is m-phenylene, p-phenylene, diarylsulfone, a group of the formula

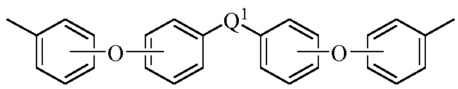

wherein $Q^1$ is a single bond, -O-, -S-, -C(O)-, $-SO_2-$, -SO-, $-C_yH_{2y}$ wherein y is an integer from 1 to 5, or a combination comprising at least one of the foregoing.

11. The method of claim 1, wherein
X is chloro, and
R is m-phenylene, p-phenylene, arylene ether, or diarylsulfone, and

the stoichiometric molar ratio of the substituted phthalic anhydride to the organic diamine is 1.98:1 to 2.02:1.

**12.** The method of Claim 11, wherein

$$\frac{\text{weight of bis(phthalimide)}}{\text{weight of bis(phthalimide)} + \text{weight of diphenyl sulphone or sulfolane}} (100)$$

is 1 to 30%, specifically 15 to 25%, preferably 18 to 22%.

**13.** A composition for manufacturing a bis(phthalimide) composition, comprising
a substituted phthalic anhydride and an organic diamine in sulfolane,
wherein the stoichiometric molar ratio of the substituted phthalic anhydride to the organic diamine is 1.95:1 to 2.05:1,
the substituted phthalic anhydride has a formula

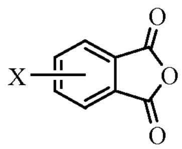

and
the organic diamine has a formula

$$H_2N\text{-}R\text{-}NH_2;$$

wherein, in the foregoing formulae,

X is fluoro, chloro, bromo, iodo, nitro, or a combination comprising at least one of the foregoing, and
R is an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, $-(C_6H_{10})_z-$ wherein z is an integer from 1 to 4, an aromatic hydrocarbyl moiety having from 1 to 6 aromatic groups, a divalent group of the formula

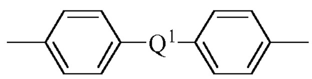

wherein $Q^1$ is a single bond, -O-, -S-, -C(O)-, $-SO_2-$, -SO-, $-C_yH_{2y}$ wherein y is an integer from 1 to 5, or a combination comprising at least one of the foregoing.

## Patentansprüche

**1.** Verfahren zur Herstellung einer Bis(phthalimid)-Zusammensetzung, wobei das Verfahren das Inkontaktbringen eines substituierten Phthalsäureanhydrids mit einem organischen Diamin in Gegenwart von Diphenylsulfon, Sulfolan oder einer mindestens eines der vorangehenden Lösungsmittel umfassenden Kombination bei einer Temperatur von mehr als 130 °C umfasst; wobei das Inkontaktbringen in Abwesenheit eines Imidisierungskatalysators durchgeführt wird;

wobei das substituierte Phthalsäureanhydrid eine Formel

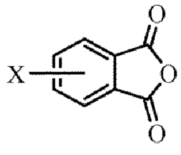

aufweist
und das organische Diamin eine Formel

$$H_2N\text{-}R\text{-}NH_2$$

aufweist;

um die Bis(phthalimid)-Zusammensetzung zu bilden, die Diphenylsulfon, Sulfolan oder eine mindestens eines der vorangehenden Lösungsmittel umfassende Kombination und ein Bis(phthalimid) der Formel

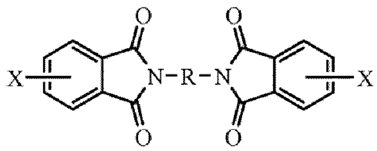

umfasst,
wobei in den vorangehenden Formeln

X für Fluor, Chlor, Brom, Iod, Nitro oder eine mindestens eines der Vorangehenden umfassende Kombination steht und
R für Folgendes steht: eine aromatische Kohlenwasserstoffgruppe mit 6 bis 27 Kohlenstoffatomen, ein halogeniertes Derivat davon, eine gerad- oder verzweigtkettige Alkylengruppe mit 2 bis 10 Kohlenstoffatomen, ein halogeniertes Derivat davon, eine Cycloalkylengruppe mit 3 bis 20 Kohlenstoffatomen, ein halogeniertes Derivat davon, $-(C_6H_{10})_z-$, wobei z für eine ganze Zahl von 1 bis 4 steht, einen aromatischen Hydrocarbylrest mit 1 bis 6 aromatischen Gruppen, eine zweiwertige Gruppe der Formel

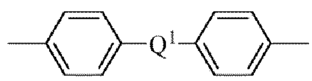
,

wobei $Q^1$ für eine Einfachbindung, -O-, -S-, -C(O)-, $-SO_2-$, -SO-, $-C_yH_{2y}-$, wobei y für eine ganze Zahl von 1 bis 5 steht, oder eine mindestens eines der Vorangehenden umfassende Kombination steht,
wobei das stöchiometrische Molverhältnis des substituierten Phthalsäureanhydrids zu dem organischen Diamin 1,95:1 bis 2,05:1 beträgt.

2. Verfahren nach Anspruch 1, wobei das Verfahren das Inkontaktbringen des substituierten Phthalsäureanhydrids und des organischen Diamins in Gegenwart von Sulfolan bei einer Temperatur von mehr als 130 °C umfasst, um die Bis(phthalimid)-Zusammensetzung, die Sulfolan und das Bis(phthalimid) umfasst, zu bilden.

3. Verfahren nach Anspruch 1 oder Anspruch 2, wobei das stöchiometrische Molverhältnis des substituierten Phthalsäureanhydrids zu dem organischen Diamin 1,98:1 bis 2,02:1 beträgt.

4. Verfahren nach irgendeinem oder mehreren der Ansprüche 1 bis 3, wobei das

$$\frac{\text{Gewicht des Bis(phthalimids)}}{\text{Gewicht des Bis(phthalimids) + Gewicht des Diphenylsulfons oder Sulfolans}} (100)$$

1 bis 30 %, insbesondere 15 bis 25 %, vorzugsweise 18 bis 22 % beträgt.

5. Verfahren nach irgendeinem oder mehreren der Ansprüche 1 bis 4, ferner umfassend
das Ermitteln des stöchiometrischen Molverhältnisses des substituierten Phthalsäureanhydrids zu dem organischen Diamin während der Imidisierung des substituierten Phthalsäureanhydrids und des organischen Diamins; und

gegebenenfalls das Einstellen des stöchiometrischen Molverhältnisses durch Zusetzen von zusätzlichem substituiertem Phthalsäureanhydrid oder organischem Diamin.

**6.** Verfahren nach irgendeinem oder mehreren der Ansprüche 1 und 3 bis 5, ferner umfassend das Erwärmen des Diphenylsulfons auf eine Temperatur von mehr als 130 °C und das Kombinieren des substituierten Phthalsäureanhydrids und des organischen Diamins mit dem erwärmten Diphenylsulfon.

**7.** Verfahren nach irgendeinem oder mehreren der Ansprüche 1 bis 6, ferner umfassend das Zusetzen eines monofunktionellen Reaktanten zu dem substituierten Phthalsäureanhydrid, dem organischen Diamin, einem Lösungsmittel oder einer mindestens eines der Vorangehenden umfassenden Kombination, wobei das Lösungsmittel Diphenylsulfon, Sulfolan oder eine mindestens eines der Vorangehenden umfassende Kombination umfasst.

**8.** Verfahren nach irgendeinem oder mehreren der Ansprüche 1 bis 7, wobei das Inkontaktbringen bei einer Temperatur von 130 °C bis 250 °C durchgeführt wird.

**9.** Verfahren nach irgendeinem oder mehreren der Ansprüche 1 bis 8, wobei X für Chlor, Fluor, Brom oder Nitro steht und R für einen zweiwertigen Rest der Formel $-(C_6H_{10})_z-$, wobei z für eine ganze Zahl von 1 bis 4 steht,

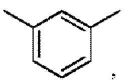 , 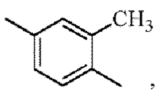 , 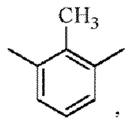 , 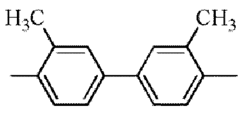 , 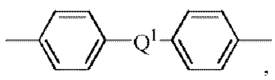 ,

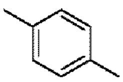 , 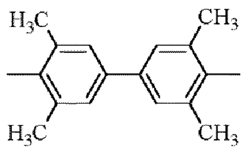 , 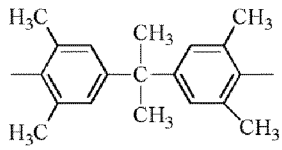 , 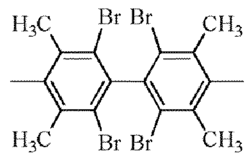 ,

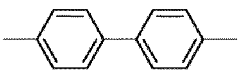 , 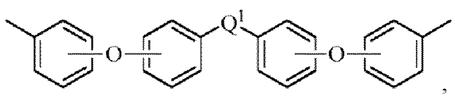 , 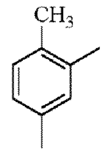

oder eine mindestens eines der Vorangehenden umfassende Kombination steht, wobei $Q^1$ für eine Einfachbindung, -O-, -S-, -C(O)-, $-SO_2-$, -SO-, $-C_yH_{2y}-$, wobei y für eine ganze Zahl von 1 bis 5 steht, oder eine mindestens eines der Vorangehenden umfassende Kombination steht.

**10.** Verfahren nach irgendeinem oder mehreren der Ansprüche 1 bis 9, wobei X für Chlor steht und R für m-Phenylen, p-Phenylen, Diarylsulfon, eine Gruppe der Formel

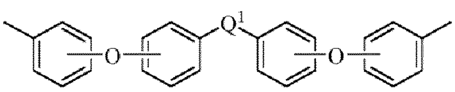

steht, wobei $Q^1$ für eine Einfachbindung, -O-, -S-, -C(O)-, $-SO_2-$, -SO-, $-C_yH_{2y}-$, wobei y für eine ganze Zahl von 1 bis 5 steht, oder eine mindestens eines der Vorangehenden umfassende Kombination steht.

**11.** Verfahren nach Anspruch 1, wobei
X für Chlor steht und
R für m-Phenylen, p-Phenylen, Arylenether oder Diarylsulfon steht und das stöchiometrische Molverhältnis des substituierten Phthalsäureanhydrids zu dem organischen Diamin 1,98:1 bis 2,02:1 beträgt.

**12.** Verfahren nach Anspruch 11, wobei
das

$$\frac{\text{Gewicht des Bis(phthalimids)}}{\text{Gewicht des Bis(phthalimids) + Gewicht des Diphenylsulfons oder Sulfolans}} (100)$$

1 bis 30 %, insbesondere 15 bis 25 %, vorzugsweise 18 bis 22 % beträgt.

**13.** Zusammensetzung zur Herstellung einer Bis(phthalimid)-Zusammensetzung, die ein substituiertes Phthalsäureanhydrid und ein organisches Diamin in Sulfolan umfasst, wobei das stöchiometrische Molverhältnis des substituierten Phthalsäureanhydrids zu dem organischen Diamin 1,95:1 bis 2,05:1 beträgt,

das substituierte Phthalsäureanhydrid eine Formel

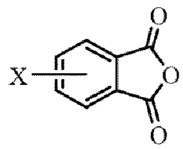

aufweist und
das organische Diamin eine Formel

$$H_2N\text{-}R\text{-}NH_2$$

aufweist;

wobei in den vorangehenden Formeln
X für Fluor, Chlor, Brom, Iod, Nitro oder eine mindestens eines der Vorangehenden umfassende Kombination steht und
R für Folgendes steht: eine aromatische Kohlenwasserstoffgruppe mit 6 bis 27 Kohlenstoffatomen, ein halogeniertes Derivat davon, eine gerad- oder verzweigtkettige Alkylengruppe mit 2 bis 10 Kohlenstoffatomen, ein halogeniertes Derivat davon, eine Cycloalkylengruppe mit 3 bis 20 Kohlenstoffatomen, ein halogeniertes Derivat davon, $-(C_6H_{10})_z-$, wobei z für eine ganze Zahl von 1 bis 4 steht, einen aromatischen Hydrocarbylrest mit 1 bis 6 aromatischen Gruppen, eine zweiwertige Gruppe der Formel

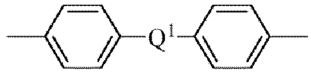
,

wobei $Q^1$ für eine Einfachbindung, -O-, -S-, -C(O)-, $-SO_2-$, -SO-, $-C_yH_{2y}-$, wobei y für eine ganze Zahl von 1 bis 5 steht, oder eine mindestens eines der Vorangehenden umfassende Kombination steht.

## Revendications

**1.** Procédé pour la fabrication d’une composition de bis(phtalimide), le procédé comprenant la mise en contact d’un anhydride phtalique substitué avec une diamine organique en présence de diphénylsulfone, de sulfolane ou d’une combinaison comprenant au moins l’un des solvants précédents à une température supérieure à 130 °C ; la mise en contact étant effectuée en l’absence de catalyseur d’imidation ;
dans lequel l’anhydride phtalique substitué a pour formule

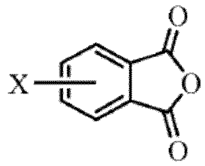

et la diamine organique a pour formule

$H_2N\text{-}R\text{-}NH_2$ ;

pour fournir la composition de bis(phtalimide) comprenant de la diphénylsulfone, du sulfolane ou une combinaison comprenant au moins l'un des solvants précédents, et un bis(phtalimide) de formule

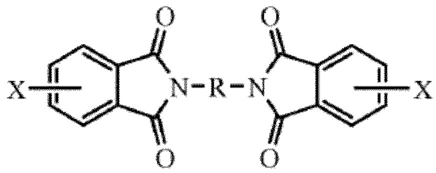

dans lequel, dans les formules précédentes,

X est un fluoro, un chloro, un bromo, un iodo, un nitro ou une combinaison comprenant au moins l'un des éléments qui précèdent, et
R est un groupe hydrocarboné aromatique ayant 6 à 27 atomes de carbone, un dérivé halogéné de celui-ci, un groupe alkylène à chaîne droite ou ramifiée ayant 2 à 10 atomes de carbone, un dérivé halogéné de celui-ci, un groupe cycloalkylène ayant 3 à 20 atomes de carbone, un dérivé halogéné de celui-ci, - $(C_6H_{10})_z$- dans lequel z est un nombre entier de 1 à 4, une fraction hydrocarbyle aromatique ayant de 1 à 6 groupes aromatiques, un groupe divalent de formule

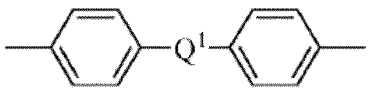

dans laquelle $Q^1$ est une simple liaison, -O-, -S-, -C(O)-, -$SO_2$-, -SO-, -$C_yH_{2y}$ dans lequel y est un nombre entier de 1 à 5, ou une combinaison comprenant au moins l'un des éléments qui précèdent,
dans lequel le rapport molaire stœchiométrique de l'anhydride phtalique substitué à la diamine organique est compris entre 1,95:1 et 2,05:1.

2. Procédé selon la revendication 1, dans lequel le procédé comprend la mise en contact de l'anhydride phtalique substitué et de la diamine organique en présence de sulfolane à une température supérieure à 130 °C pour donner la composition de bis(phtalimide) comprenant du sulfolane et du bis(phtalimide).

3. Procédé selon la revendication 1 ou la revendication 2, dans lequel le rapport molaire stœchiométrique de l'anhydride phtalique substitué à la diamine organique est compris entre 1,98:1 et 2,02:1.

4. Procédé selon l'une quelconque ou plusieurs des revendications 1 à 3, dans lequel

$$\frac{poids\ de\ bis(phtalimide)}{poids\ de\ bis(phtalimide) +\ poids\ de\ diphénylsulfone\ ou\ de\ sulfolane}(100)$$

est de 1 à 30 %, plus précisément de 15 à 25 %, de préférence de 18 à 22 %.

5. Procédé selon l'une quelconque ou plusieurs des revendications 1 à 4, comprenant en outre
la détermination du rapport molaire stœchiométrique de l'anhydride phtalique substitué à la diamine organique pendant l'imidation de l'anhydride phtalique substitué et de la diamine organique ; et

éventuellement l'ajustement du rapport molaire stœchiométrique par ajout d'anhydride phtalique substitué ou de diamine organique supplémentaire.

6. Procédé selon l'une quelconque ou plusieurs des revendications 1, et 3 à 5, comprenant en outre le chauffage de la diphénylsulfone à une température supérieure à 130 °C, et la combinaison de l'anhydride phtalique substitué et de la diamine organique avec la diphénylsulfone chauffée.

7. Procédé selon l'une quelconque ou plusieurs des revendications 1 à 6, comprenant en outre l'ajout d'un réactif monofonctionnel à l'anhydride phtalique substitué, à la diamine organique, à un solvant ou à une combinaison comprenant au moins l'un des éléments qui précèdent, dans lequel le solvant comprend de la diphénylsulfone, du sulfolane ou une combinaison comprenant au moins l'un des éléments qui précèdent.

8. Procédé selon l'une quelconque des revendications 1 à 7, dans lequel la mise en contact est effectuée à une température de 130 °C à 250 °C.

9. Procédé selon l'une quelconque ou plusieurs des revendications 1 à 8, dans lequel
X est un chloro, un fluoro, un bromo ou un nitro, et
R est un radical divalent de formule $-(C_6H_{10})_z-$ dans laquelle z est un nombre entier de 1 à 4,

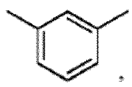 , 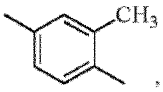 , 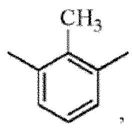 , 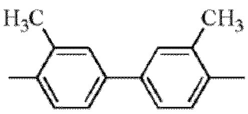 , 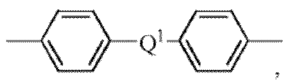 ,

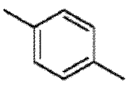 , 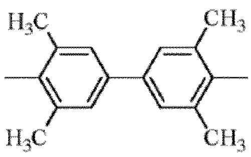 , 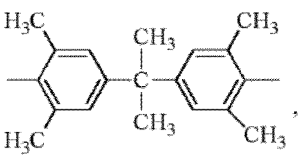 , 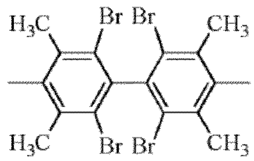 ,

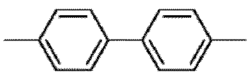 , 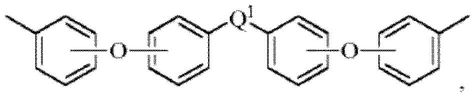 , 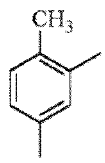

ou une combinaison comprenant au moins l'un des éléments qui précèdent, dans lequel $Q^1$ est une simple liaison, -O-, -S-, -C(O)-, $-SO_2-$, -SO-, $-C_yH_{2y}-$ dans lequel y est un nombre entier de 1 à 5, ou une combinaison comprenant au moins l'un des éléments qui précèdent.

10. Procédé selon l'une quelconque ou plusieurs des revendications 1 à 9, dans lequel X est un chloro et R est un m-phénylène, un p-phénylène, une diarylsulfone, un groupe de formule

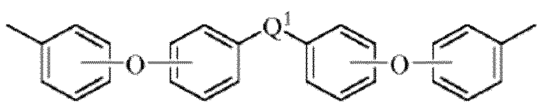

dans laquelle $Q^1$ est une simple liaison, -O-, -S-, -C(O)-, $-SO_2-$, -SO-, $-C_yH_{2y}$ dans lequel y est un nombre entier de 1 à 5, ou une combinaison comprenant au moins l'un des éléments qui précèdent.

11. Procédé selon la revendication 1, dans lequel
X est un chloro, et
R est un m-phénylène, un p-phénylène, un arylène éther ou une diarylsulfone, et
le rapport molaire stœchiométrique de l'anhydride phtalique substitué à la diamine organique est compris entre

1,98:1 et 2,02:1.

**12.** Procédé selon la revendication 11, dans lequel

$$\frac{poids\ de\ bis(phtalimide)}{poids\ de\ bis(phtalimide) + \ poids\ de\ diphénylsulfone\ ou\ de\ sulfolane}(100)$$

est de 1 à 30 %, plus précisément de 15 à 25 %, de préférence de 18 à 22 %.

**13.** Composition pour la fabrication d'une composition de bis(phtalimide), comprenant un anhydride phtalique substitué et une diamine organique dans du sulfolane,
dans laquelle le rapport molaire stœchiométrique de l'anhydride phtalique substitué à la diamine organique est compris entre 1,95:1 et 2,05:1,
l'anhydride phtalique substitué a pour formule

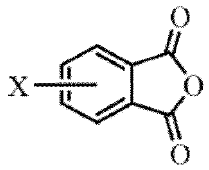

et
la diamine organique a pour formule

$H_2N\text{-}R\text{-}NH_2$ ;

dans laquelle, dans les formules précédentes,

X est un fluoro, un chloro, un bromo, un iodo, un nitro ou une combinaison comprenant au moins l'un des éléments qui précèdent, et
R est un groupe hydrocarboné aromatique ayant 6 à 27 atomes de carbone, un dérivé halogéné de celui-ci, un groupe alkylène à chaîne droite ou ramifiée ayant 2 à 10 atomes de carbone, un dérivé halogéné de celui-ci, un groupe cycloalkylène ayant 3 à 20 atomes de carbone, un dérivé halogéné de celui-ci, - $(C_6H_{10})_z$- dans lequel z est un nombre entier de 1 à 4, une fraction hydrocarbyle aromatique ayant de 1 à 6 groupes aromatiques, un groupe divalent de formule

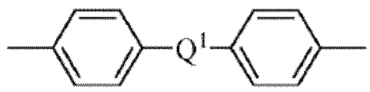

dans laquelle $Q^1$ est une simple liaison, -O-, -S-, -C(O)-, -$SO_2$-, -SO-, -$C_yH_{2y}$ dans lequel y est un nombre entier de 1 à 5, ou une combinaison comprenant au moins l'un des éléments qui précèdent.

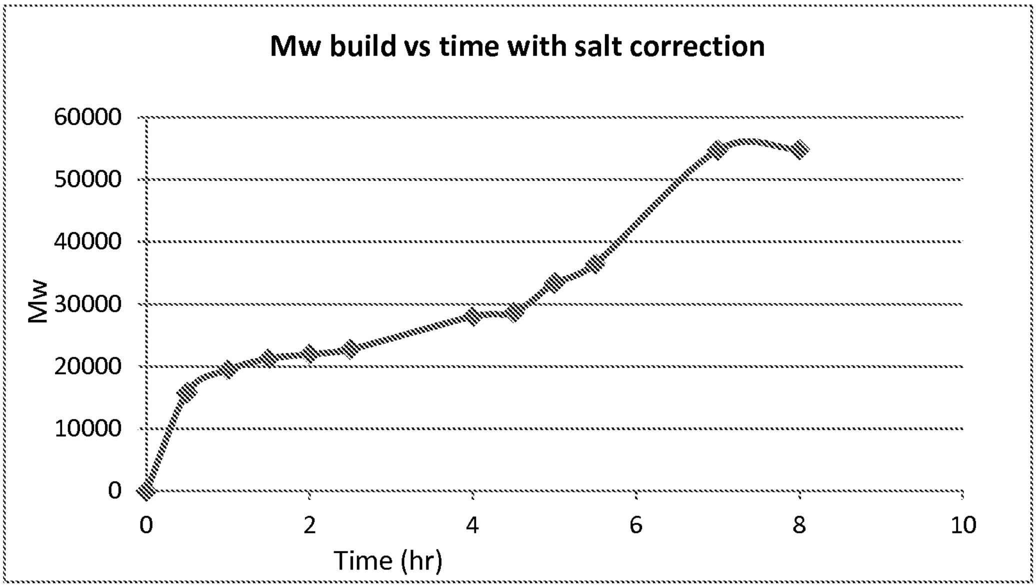
Mw build vs time with salt correction
60000
50000
40000
30000
20000
10000
0
Mw
0
2
4
6
8
10
Time (hr)

FIG. 1

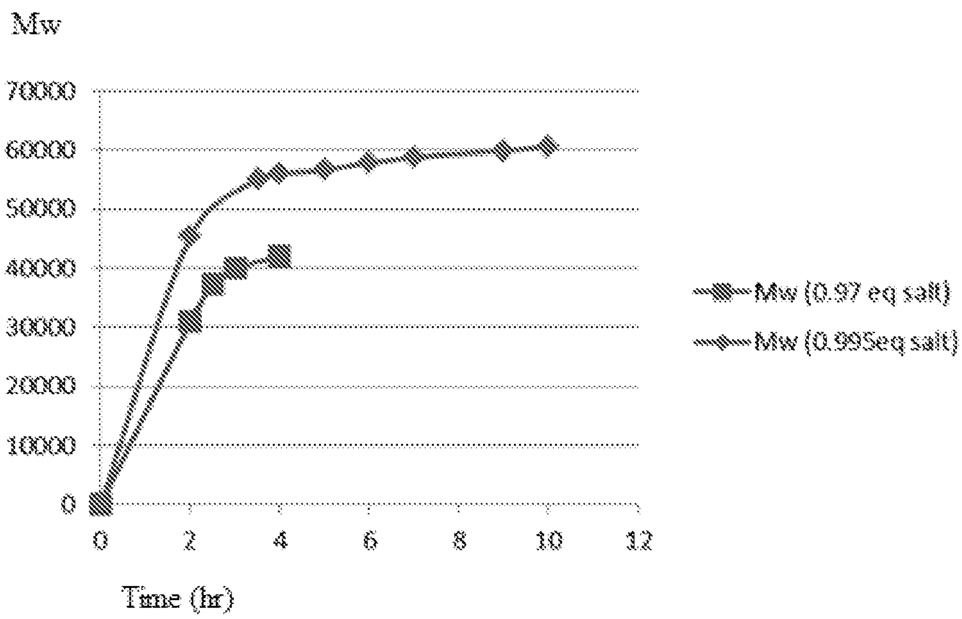
Mw
70000
60000
50000
40000
30000
20000
10000
0
0
2
4
6
8
10
12
Time (hr)
Mw (0.97 eq salt)
Mw (0.995eq salt)

FIG. 2